# EUROPÄISCHE PATENTSCHRIFT

(11) **EP 0 781 532 B1**
(45) Veröffentlichungstag und Bekanntmachung des Hinweises auf die Patenterteilung: **20.03.2002**
(21) Anmeldenummer: 96119232.5
(22) Anmeldetag: 29.11.1996
(51) Int. Cl.: A61F 2/30, A61N 1/04

(54) **Implantierbare Vorrichtung mit Innenelektrode**
Implantable device with inner electrode
Dispositif pour implantation avec une électrode interne

(30) Priorität: 30.11.1995 DE 19544750
(43) Veröffentlichungstag der Anmeldung: 02.07.1997
(73) Patentinhaber: Rehberg, Christoph, 80796 München (DE)
(72) Erfinder: Rehberg, Christoph, D - 80796 München (DE); Kraus, Werner, D-80333 München (DE)
(74) Vertreter: Hertz, Oliver, Dr.

(56) Entgegenhaltungen:
- DE-A- 3 709 734
- DE-A- 19 508 753

## Beschreibung

Die vorliegende Erfindung betrifft eine implantierbare Vorrichtung mit einem ihrer Funktion entsprechenden, aus biokompatiblem Material bestehenden Körper, welcher einen inneren Hohlraum bildet, mindestens eine Durchbrechung aufweist und mit Elektroden versehen ist, denen eine niederfrequente Wechselspannung zuführbar ist.

In den menschlichen Körper implantierbare Vorrichtungen der hier interessierenden Art, wie Gelenkprothesen, Zahnprothesen, Knorpelsubstrate, z. B. zum Aufbau von Ohrgewebe, u. a. m. haben einen ihrer medizinischen Funktion entsprechenden, lasttragenden oder formgebenden Körper aus einem biokompatiblen Material, wie Metall, Kunststoff oder Keramik.

Aus der Zeitschrift OSTEOLOGIE, Band 5, Heft 2, 1996, S. 87 sind Gelenkprothesen aus Titan bekannt, die durch Spreizhülsen am Knochen befestigt werden.

Aus den "Orthopedic Product News" May/June 1995, S. 8 sind Implantate zur Stabilisierung von Rückenwirbeln bekannt.

Es sind ferner implantierbare Vorrichtung bekannt, deren Körper einen Hohlraum bildet und offene Durchbrechungen aufweist, die vom Hohlraum nach außen führen. Der Hohlraum dient zur Aufnahme von Spongiosa oder anderem bioaktiven Material, das das Einwachsen des die implantierte Vorrichtung umgebenden Gewebes in die Durchbrechungen fördern soll. Ein typisches Beispiel einer solchen Vorrichtung ist die von Prof. Täger entwickelte Hüftgelenkprothese. Der Hüftgelenkkopfteil dieser Prothese hat einen hohlen, mit seitlichen Durchbrechungen versehenen Schaft. Der Gelenkpfannenteil ist doppelwandig und bildet einen ringförmigen Hohlraum. Die Außenwand bildet eine Art von Außengewinde und hat eine Reihe schräger, schlitzförmiger Durchbrechungen.

Es ist auch bekannt, daß das Wachstum von Gewebe, insbesondere Knochengewebe, des menschlichen Körpers durch einen niederfrequenten Wechselstrom gefördert werden kann. Vorrichtungen ("Elektro-Implantate"), die von dieser Erkenntnis Gebrauch machen, enthalten eine implantierbare Aufnehmer- oder Sekundärspule, die mit Gewebeelektroden verbunden oder gekoppelt ist und in der durch eine externe Primärspule eine niederfrequente Wechselspannung induzierbar ist. Frequenzen unter 20 Hz, vorzugsweise unter 15 oder 10 Hz haben sich bewährt, die Kurvenform soll sinus- oder pulswellenförmig sein. Dieses Prinzip findet auch bei der vorliegenden Erfindung Anwendung.

Eine aus der DE-C-23 15 517 bekannte Elektro-Hüftkopfprothese hat einen Schaft mit einem Hohlraum, in dem eine Aufnehmerspule untergebracht ist. Die Aufnehmerspule ist mit Elektroden gekoppelt, die außen auf dem Schaft angebracht sind.

Eine Elektro-Zahnprothese in Form eines Kunstzahnes aus Keramik, der eine Aufnehmerspule enthält und auf seiner Außenfläche Elektroden trägt, ist aus der DE-C-26 11 744 bekannt.

Eine Vorrichtung zur Vitalerhaltung von Knochengewebe mit einer Aufnehmerspule und zwei mit dieser verbundenen Elektoden, die in einen kegelstumpfmantelförmigen netzartigen Träger integriert sind, ist aus der DE-A-30 03 758 bekannt.

Ein Marknagel, der eine Aufnehmerspule enthält und auf der Außenseite zwei Gewebeelektroden trägt, ist aus der US-A-38 20 534 bekannt.

Aus der DE-A-34 14 992 ist ein künstlicher Zahn bekannt, der einen Schaft aus gewebeverträglichem Metall hat. Der Schaft hat eine rechteckige, diametrale Durchbrechung, an deren Innenwand eine Elektrode angeordnet ist. Zwischen den Schaft und die Elektrode kann eine niederfrequente Wechselspannung angelegt werden, um das Einwachsen des Schaftes in den Kiefernknochen zu fördern. Der das Gewebewachstum fördernde Strom fließt im wesentlichen von den Außenrändern der in der Durchbrechung angeordneten Elektrode zu den die Durchbrechung begrenzenden Rändern des Schaftes, nicht jedoch innerhalb der Durchbrechung, so daß das Gewebe nicht veranlaßt wird in die Durchbrechung hinein zu wachsen.

Der vorliegenden Erfindung liegt die Aufgabe zugrunde eine implantierbare Vorrichtung der eingangs genannten Art so auszugestalten, daß das Einwachsen des äußeren Gewebes in den Hohlraum oder die Hohlräume der Vorrichtung gefördert wird, also eine Vorrichtung, die einen ihrer medizinischen Funktion entsprechend gestalteten, lasttragenden oder formgebenden Körper aus biokompatiblen Material hat, der mindestens einen inneren Hohlraum bildet, in den, wenn gewünscht, Spongiosa oder anderes biologisch wirksames Material eingebracht werden kann, wobei vom Hohlraum Durchbrechungen oder Öffnungen nach Außen führen, durch die das Gewebe, das die implantierte Vorrichtung umgibt, in den Hohlraum hinein wachsen soll.

Diese Aufgabe wird erfindungsgemäß dadurch gelöst, daß die Vorrichtung in bekannter Weise mit einer Aufnehmerspule oder anderen Stromzuführungsvorrichtung versehen ist, die mit mindestens zwei Elektroden gekoppelt ist, und daß mindestens eine der Elektroden innerhalb des Hohlraumes so angeordnet ist, daß sie von der inneren Begrenzung des Hohlraumes mit Abstand umgeben wird.

Der Bereich des Hohlraumes zwischen mindestens einer Durchbrechung, die im implantierten Zustand der Vorrichtung zum umgebenden, zum Einwachsen zu veranlassenden Gewebe hin weist, und der innerhalb des Hohlraums angeordneten Elektrode ist ganz oder teilweise frei von Teilen der Vorrichtung, so daß er biologisch aktives Material und/oder einwachsendes Gewebe aufnehmen kann. Wenn den Elektroden eine niederfrequente Wechselspannung zugeführt wird, entsteht ein von innen nach außen gerichtetes niederfrequentes elektrisches Wechselfeld und fließt ein niederfrequenter Wechselstrom im Inneren des Hohlraumes, die das Einwachsen des äußeren Gewebes durch die Durchbrechungen oder Löcher in den Hohlraum fördern und dadurch das Implantat im umgebenden Gewebe fest verankern.

Wenn hier von Durchbrechungen oder Löchern gesprochen wird, sind makroskopische Löcher gemeint, nicht mikroskopische Poren, wie sie für feinporige Sintermaterialien typisch sind. Bei starren Implantaten werden die Durchbrechungen im allgemeinen Querschnittsflächen im Bereich von einigen bis mehreren 10mm² und mehr haben. Bei grobporigen, gesinterten oder netzartigen Strukturen haben die Durchbrechungen im allgemeinen Größen im Bereich von einigen Zehntel mm² bis zu einigen mm².

Der lasttragende oder formgebende Körper kann die verschiedenste Gestalt haben, z. B. die einer Hüftgelenkkopfprothese, einer Hüftgelenkpfannenprothese oder anderer Gelenkprothesen, einer Verankerung für eine Zahnprothese, eines Lendenwirbelsäulen-Plugs oder einer formgebenden netzartigen Struktur für den Aufbau einer Ohrmuschel, Nase u. s. w.

Wenn der durchbrochene Körper elektrisch leitfähig und als Gegenelektrode zu der in seinem Innenraum isoliert von ihm angeordneten Innenelektrode geschaltet ist, kann es vorteilhaft sein, zwischen die Elektroden eine niederfrequente Wechselspannung zu legen, der eine Gleichstromkomponente solcher Polarität überlagert ist, daß die äußere Elektrode die Kathode bildet. Die Größe der Gleichspannungskomponente kann z. B. 20%, ggf. bis zu 50% der gewöhnlich bis zu etwa 700 mV/eff betragenden Amplitude der Wechselspannung ausmachen. Bei Verwendung einer implantierten Aufnehmerspule als Spannungsquelle kann die Gleichspannungskomponente dadurch erzeugt werden, daß man in die Leitung zwischen einer der Elektroden und dem zugehörigen Anschluß der Aufnehmerspule eine Diode schaltet, der ein Widerstand parallel liegt.

In folgenden wird die Erfindung anhand von Ausführungsbeispielen unter Bezugnahme auf die Zeichnungen näher erläutert. Die Abbildungen sind zum Teil stark vergrößert.

Es zeigen:
- Fig. 1: eine Schnittansicht einer bekannten Hüftgelenkopfprothese und eine für diese bestimmte Elektrifizieroder Zusatzvorrichtung gemäß der Erfindung;
- Fig. 2: eine etwas abgewandelte Ausführungsform der Elektrifiziervorrichtung gemäß Fig. 1, die eine bevorzugten inneren Aufbau solcher Vorrichtungen erkennen läßt,
- Fig. 3: eine Prinzipdarstellung einer weiteren Ausführungsform der Erfindung,
- Fig. 4a: eine Ausführungsform der Erfindung, die sich insbesondere für ein dauernd implantiertes Osteosyntheseelement eignet,
- Fig. 4b: eine Armgelenkprothese entsprechend Fig. 4a,
- Fig. 5 und 6: zwei Ausführungsbeispiele in Form von Dentalimplantaten,
- Fig. 7a: eine teilweise geschnittene, perspektivische Ansicht einer Hüftgelenkpfannenprothese gemäß der Erfindung,
- Fig. 7b: eine Draufsicht einer Elektrifiziervorrichtung der Prothese gemäß Fig. 7a,
- Fig. 7c: einen etwas vergrößerten Querschnitt in einer Ebene A - A der Fig. 7b,
- Fig. 8a: eine vereinfachte Darstellung einer anderen Ausführungsform einer Hüftgelenkpfannenprothese,
- Fig. 8b: eine Darstellung der Elektrifiziervorrichtung der Prothese gemäß Fig. 8a,
- Fig. 8c: eine Abwandlung der Vorrichtung gemäß Fig. 8b,
- Fig 8d: eine weitere Abwandlung der Vorrichtung gemäß Fig. 8b,
- Fig. 9a: eine zur Förderung des Aufbaus von Knorpel- und Bindegewebe dienende Ausführungsform,
- Fig. 9b: eine etwas vergrößerte Schnittansicht der Vorrichtung gemäß Fig 9a in einer Ebene B - B der Fig 9a,
- Fig. 10: eine Schnittansicht eines weiteren Dentalimplantats,
- Fig. 11: eine abgewandelte Aufnehmerspuleneinheit,
- Fig. 12: eine zwischen zwei Lendenwirbeln implantierte Vorrichtung,
- Fig. 13: eine Anschlußkappe für die Vorrichtung gemäß Fig. 12,
- Fig. 14a: eine anderes Beispiel eines Dentalimplantats,
- Fig. 14b: eine Zusatzvorrichtung zum Ankoppeln einer externen Spannungsquelle an das Dentalimplantat gem. Fig. 14a,
- Fig. 15: eine weitere Vorrichtung der in Fig. 12 und 13 dargestellten Art,
- Fig. 16a und 16b: Teile einer Kniegelenkprothese,
- Fig. 16c: einen Axialschnitt eines in Fig. 16b auseinandergezogen dargestellten Spreiz-Dübels,
- Fig. 17a und 17b: eine Seitenansicht bzw. Schnittansicht eines Teiles eines Marknagels,
- Fig. 17c: eine teilweise geschnittene Ansicht einer erfindungsgemäßen Vorrichtung für den Marknagel gemäß Fig 17a und 17b.
- Fig. 18a und 18b: eine geschnittene bzw. isometrische Darstellung einer Hüftgelenkschalenprothese, und
- Fig 19: eine auseinandergezogene Darstellung eines Elektro-Dübels gemäß der Erfindung.

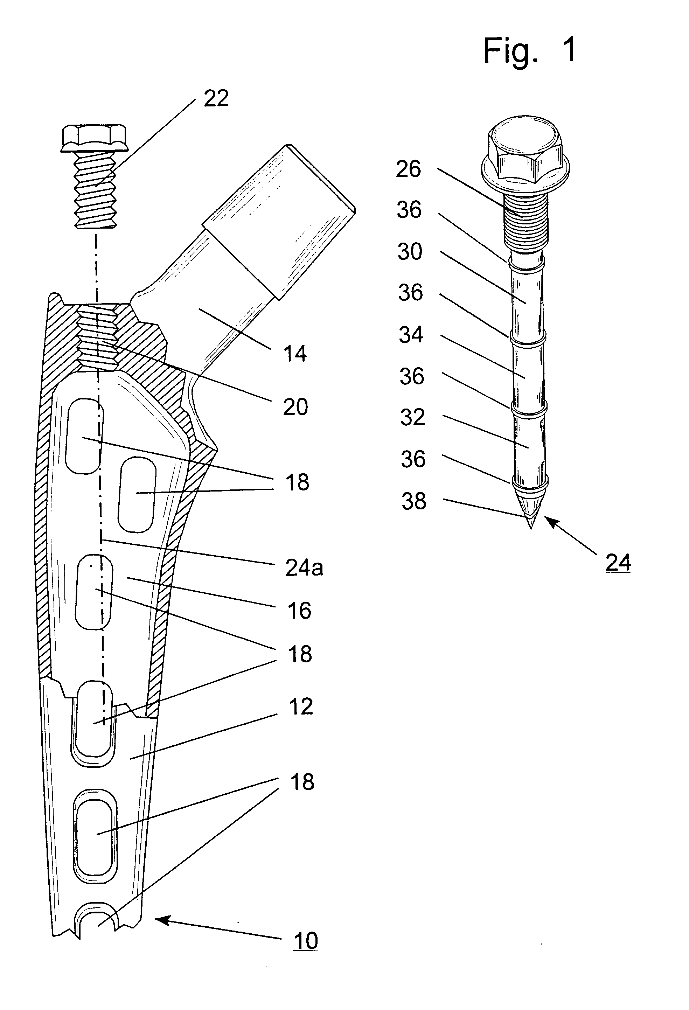

Fig. 1 zeigt links, teilweise geschnitten, eine Hüftgelenkkopfprothese 10. Diese Prothese hat einen Körper in Form eines hohlen Schaftes 12, der am oberem Ende in einen integralen Hals 14 übergeht, der zur Halterung eines Gelenkkopfes (nicht dargestellt) dient. Der Schaft 12 bildet einen Hohlraum 16, von dem aus seitliche Durchbrechungen 18 nach außen führen. Am halsseitigen Ende des Hohlraumes 16 befindet sich ein Gewindeloch 20, das zum Auffüllen des Schaftes und zum Einsetzen eines Extraktionswerkzeugs dient, es wird im bekannten Fall mit einer Einschlag-Schraube 22 verschlossen. Der Hohlraum 16 wird gewöhnlich mit Spongiosa gefüllt, in die das den implantierten Schaft umgebende Knochengewebe hineinwachsen soll. Soweit beschrieben, ist die Prothese 10 bekannt.

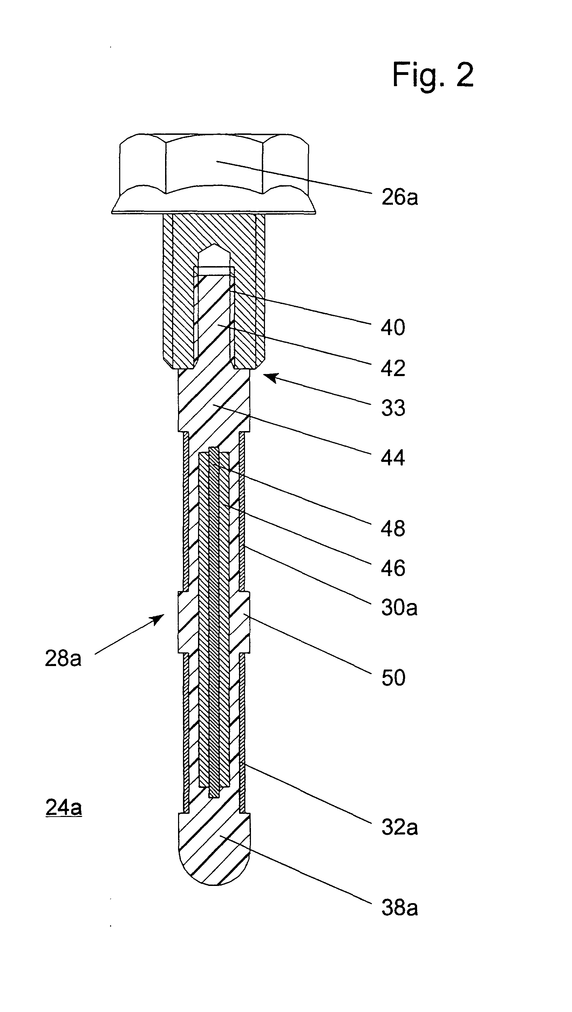

Gemäß der Erfindung wird die Schraube 22 durch eine Elektrifiziervorrichtung ersetzt, die die Prothese zu einer Elektroprothese im eingangs erläuterten Sinne macht. Die in Fig. 1 rechts beispielsweise dargestellte Vorrichtung 24, deren Konstruktion im wesentlichen der der im folgenden beschriebenen Vorrichtung gem. Fig. 2 entsprechen kann, hat einen Kopf 26 mit einem Außen- oder Innen-Sechskant, einem Flansch und einem in das Gewindeloch 20 passenden Gewinde. An den Kopf schließt sich ein stabartiger Schaft 28 an, der eine Aufnehmerspule (46 in Fig.2) enthält, die mit zwei in Längsrichtung des Schaftes beabstandeten, den Schaft umgebenden rohrförmigen Elektroden 30, 32 elektrisch gekoppelt ist. Zwischen den Elektroden 30, 32 befindet sich ein elektrisch isolierender Abschnitt 34. Der Schaft 28 ist ferner mit mehreren isolierenden ringförmigen Abstandshaltern 36 umgeben und läuft in eine als Dorn oder Bohrer ausgebildete Spitze 38 aus. Die Elektrifiziervorrichtung 24 wird anstelle der Schraube 22 eingesetzt, wie durch eine strichpunktierte Linie 24a angedeutet ist, sodaß sich der Schaft 28 im Bereich der Durchbrechungen 18 in den Hohlraum 16 erstreckt. Wenn die so ausgestaltete Prothese implantiert und der Hohlraum mit Spongiosa gefüllt ist und dann in der Aufnehmerspule mittels einer äußeren, mit einem niederfrequenten Wechselstrom gespeisten Spule eine niederfrequente Wechselspannung induziert wird, entsteht im Inneren des Hohlraumes 16 ein niederfrequentes elektrisches Wechselfeld und fließt in dem sich im Inneren des Hohlraumes 16 befindlichen Material ein niederfrequenter Wechselstrom, wodurch das Einwachsen des den Prothesenschaft 12 umgebenden Knochengewebes in die Durchbrechungen 18 gefördert und damit der Halt der Prothese gefestigt werden.

Fig. 2 zeigt im Längsschnitt eine in Bezug auf die Vorrichtung 24 der Fig. 1 etwas abgewandelte Elektrifiziervorrichtung 24a für eine Prothese gem. Fig. 1. Der aus Titan bestehende Kopf 26a hat am unteren Ende ein Innengewinde 40, in das ein als Außengewinde ausgebildetes oberes Ende 42 des Schaftes 28a eingeschraubt ist, der einen aus biokompatiblen Kunststoff, wie PTFE, bestehenden Körper 44 hat. Der Körper 44 umschließt eine Aufnehmerspule 46, deren Wicklung einen Magnetkern 48 umgibt, und trägt außen zwei in Längsrichtung des Schaftes 28a beabstandete rohrförmige Elektroden 30a, 32a, zwischen denen sich ein als Abstandshalter dienender isolierender Steg 50 befindet. Die hier abgerundete Spitze 38a des Körpers 44 ist verdickt und bildet einen weiteren Abstandshalter. Der sich an das Außengewinde anschließende Abschnitt des Schaftes 28a bildet eine Sollbruchstelle in Form einer Einschnürung 33 oder dgl., die es ermöglicht, den Kopf 26a entfernen zu können und ein Extraktionswerkzeug einzuschrauben, ohne daß der gegebenenfalls eingewachsene Schaft 28a der Elektrifiziervorrichtung aus der Prothese entfernt werden muß.

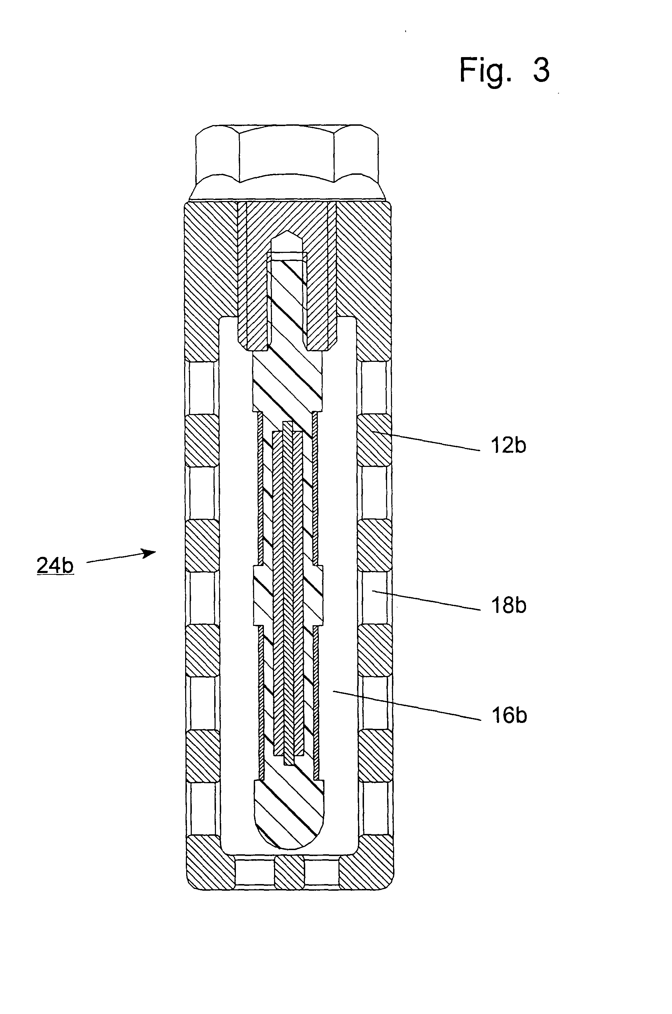

Fig. 3 zeigt eine Prinzipdarstellung einer erfindungsgemäßen Vorrichtung. Die Vorrichtung gem. Fig. 3 hat einen rohrförmig dargestellten Körper 12b, der einen Hohlraum 16b bildet und seitliche Durchbrechungen 18b aufweist. Das eine Ende des Körpers 12b weist eine Stirnwand mit Durchbrechungen auf, in das andere Ende ist eine Elektrifiziervorrichtung 24b der anhand von Fig. 2 erläuterten Art eingeschraubt. Hinsichtlich der Funktion der Vorrichtung gem. Fig. 3 gelten die unter Bezug auf Fig. 1 gemachten Ausführungen.

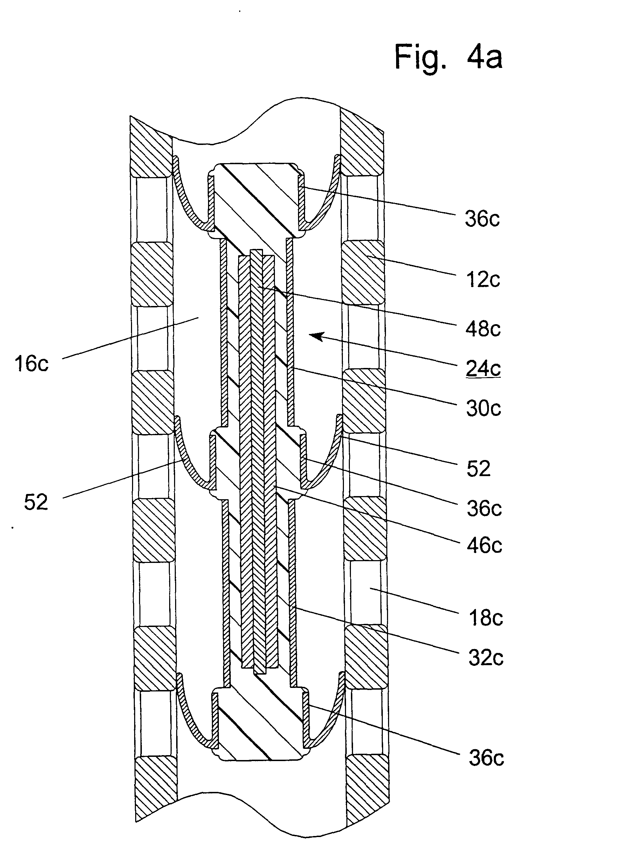

Fig. 4a zeigt eine Ausführungsform der vorliegenden Vorrichtung, die sich z. B. für dauerhaft implantierte rohrförmige Osteosynthese-Elemente oder Endoprothesen, wie Gelenkprothesenschäfte und Marknägel, eignet. Ein solches Implantat hat einen rohrförmigen Körper 12c, der einen Hohlraum 16c umschließt und seitliche Durchbrechungen 18c hat. In den Hohlraum 16c ist eine Elektrifizier-Vorrichtung 24c eingeschoben, die im Wesentlichen der der Fig. 2 entspricht, entsprechende Teile sind mit den gleichen Bezugszahlen bezeichnet, denen ein "c" angefügt ist. Sie hat jedoch keinen Schraubenkopf und wird durch ringförmige Abstandshalter 36c, die seitlich vorspringende Federbügel 52 aufweisen, im Abstand von der Innenwand des Körpers 12c gehalten. Die Funktion der Vorrichtung gem. Fig. 4a entspicht der der Fig. 1.

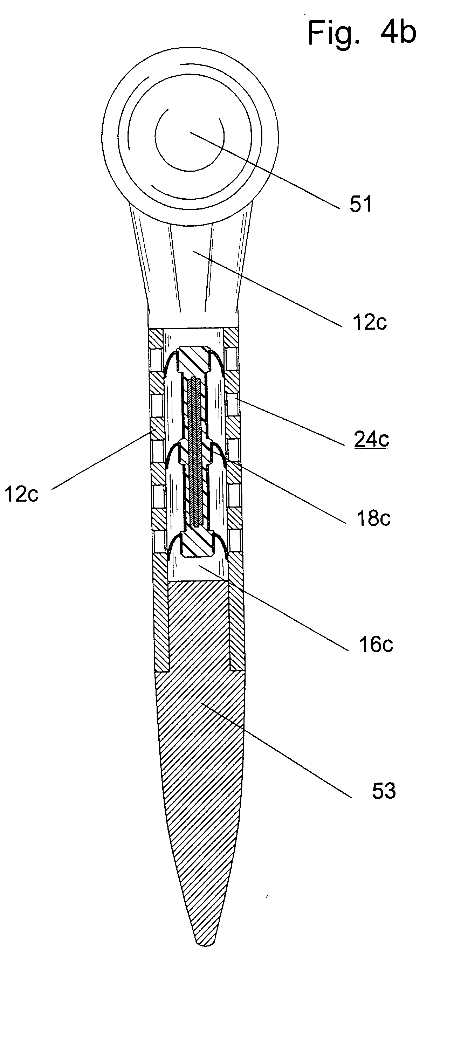

Fig. 4b zeigt eine Armgelenkprothese mit rohrförmigem Schaft 12c in den eine Elektrifiziervorrichtung 24c eingesetzt ist, wie sie anhand von Fig. 4a beschrieben wurde. Am einen Ende des Schaftes 12c befindet sich ein Gelenkkopf 51, das andere Ende ist nach dem Einsetzen der Elektrifiziervorrichtung 24c durch einen eingepreßten (oder eingeschraubten) Stopfen 53 verschlossen worden.

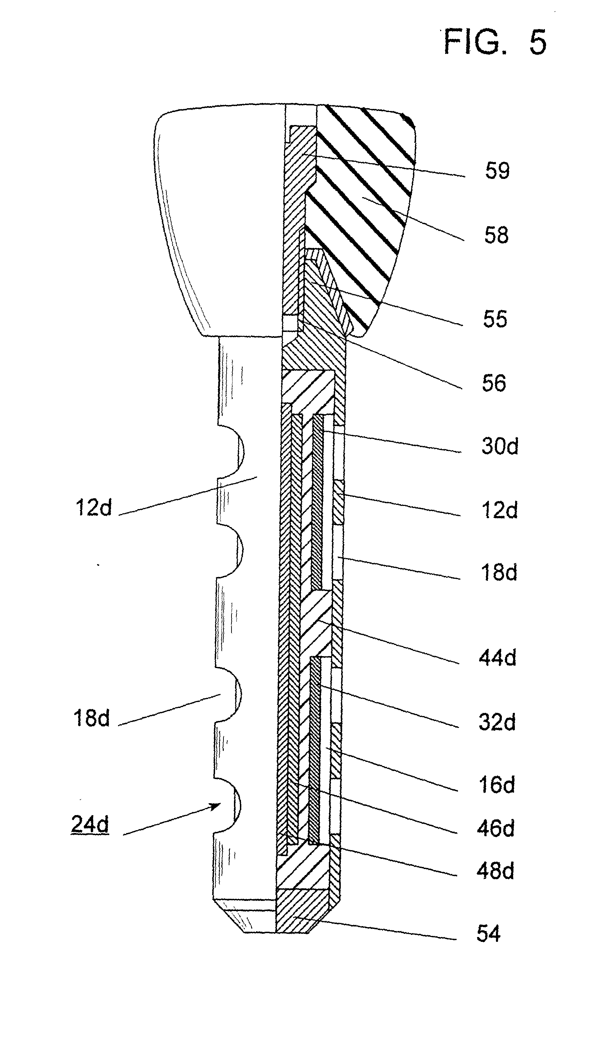

Fig. 5 zeigt eine erfindungsgemäße Vorrichtung 24d in Form eines Zahnimplantats. Sie hat einen rohrförmigen Mantel oder Körper 12d aus gewebeverträglichem Metall, wie Titan, der einen Hohlraum 16d bildet, seitliche Durchbrechungen 18d aufweist und unten durch einen eingepreßten Stopfen 54 geschlossen ist. Das andere Ende des Körpers 16d bildet einen stabilen konischen Prothesenträger 55 mit einem Innengewinde 56. Auf dem Prothesenträger 55 ist ein Kunstzahn 58 mit einer in das Innengewinde eingeschraubten Schraube 59 befestigt. Im Hohlraum 16d befindet sich eine Vorrichtung 24d der in Fig. 2 dargestellten Art mit einem Kunststoffschaft 44d, der eine Aufnehmerspule 46d mit Magnetkern 48d enthält und Elektroden 30d, 32d trägt.

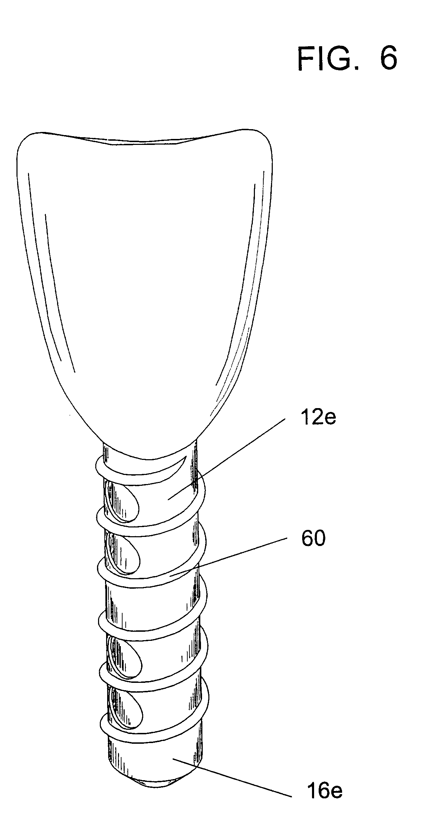

Fig. 6 zeigt eine andere Ausführungsform eines Dentalimplantats. Es entspricht dem der Fig. 5 mit der Ausnahme, daß der Körper 12e außen ein Gewinde 60 bildet, mit dem der Körper 16e in den Kieferknochen eingeschraubt werden kann.

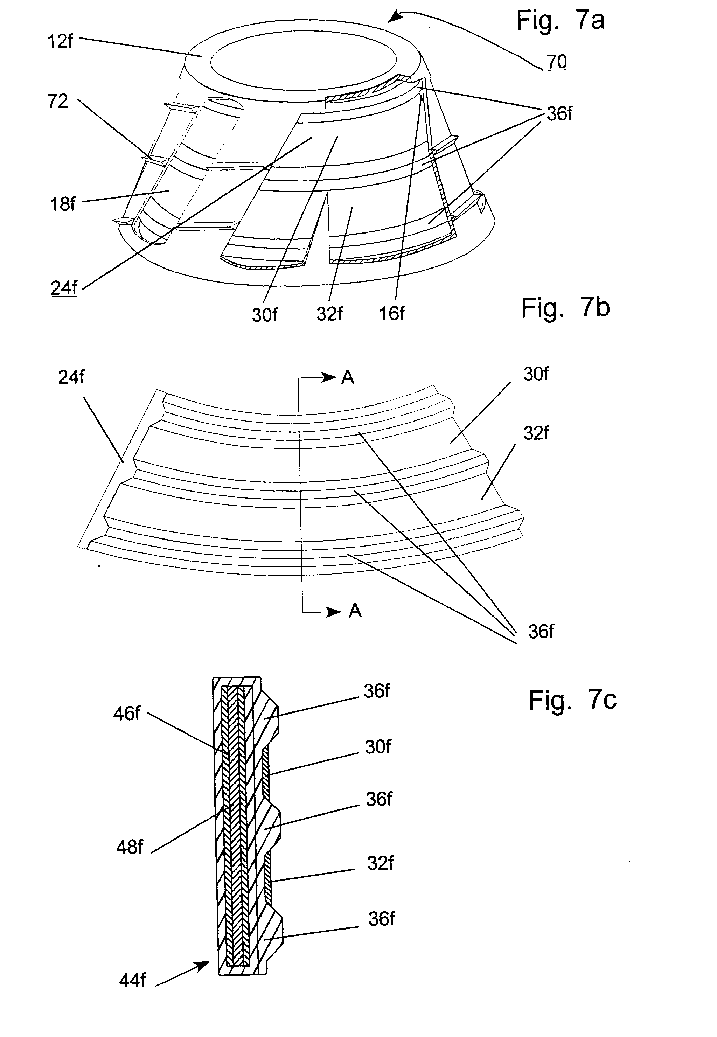

Die Fig. 7a zeigt eine bekannte Hüftgelenkpfannen-Endoprothese 70, die erfindungsgemäß mit einer in den Fig. 7b und 7c genauer dargestellten Elektrifiziervorrichtung 24f versehen ist. Die Endoprothese 70 ist doppelwandig und bildet einen ringförmigen, im wesentlichen kegelstumpfmantelförmigen Hohlraum 16f. Die Außenwand hat schräge Durchbrechungen 18f und Vorsprünge 72, die ein Gewinde zum Einschrauben in den Hüftknochen bilden.

Die Elektrifiziervorrichtung 24f hat, wie Fig. 7b und 7c zeigen, die Form eines flexiblen Bandes, das der Form des Hohlraumes 16f entsprechend kreisringsektorförmig ist und der durch eine der Durchbrechungen 18f in den Hohlraum 16f eingeführt werden kann. Sie enthält einen streifenförmigen Magnetkern 48f, der von der Wicklung einer Aufnehmerspule 46f umgeben ist. Magnetkern und Spule sind in einen Körper 44f aus einem flexiblen, biokompatiblen Material, wie PTFE, eingebettet. Der Körper trägt auf seiner einen, im eingesetzten Zustand den Durchbrechungen zugewandten Seite zwei streifenförmige Elektroden 30f, 32f, die mit den Enden der Aufnehmerspule 46f gekoppelt sind, und bildet an den Außenrändern und zwischen den Elektroden 30f, 32f drei leistenförmige Abstandshalter 36f.

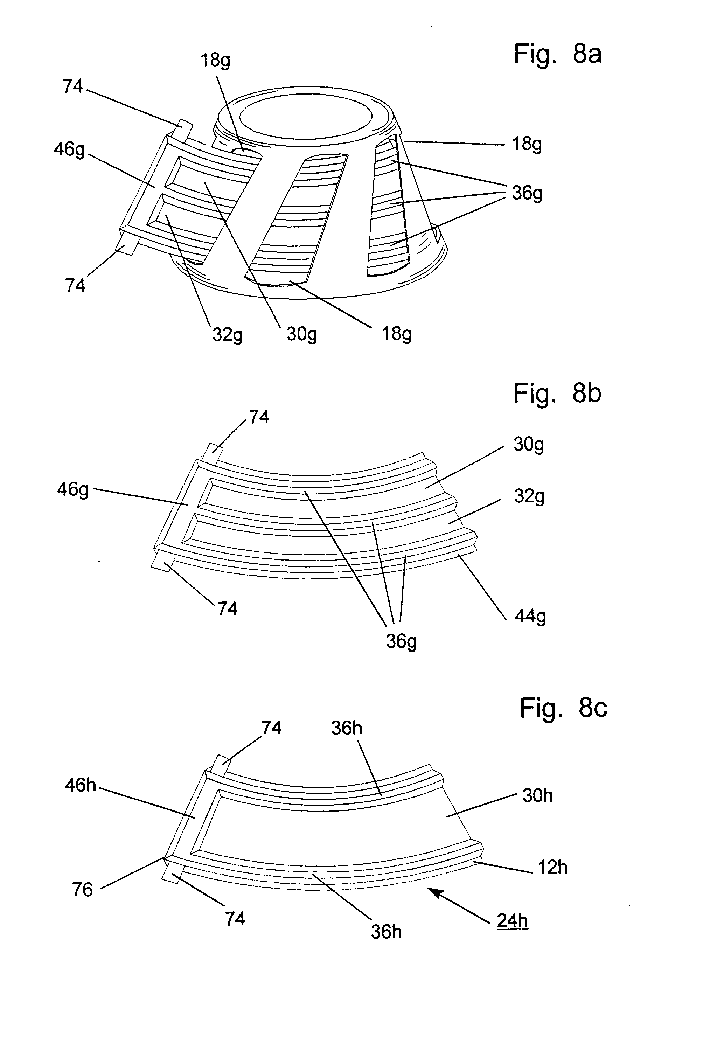

Wie die Fig. 8a und 8b zeigen, kann die Aufnehmerspule 46g bei der Elektrifiziervorrichtung für die Hüftgelenkpfannen-Prothese auch am einen Ende eines bandförmigen, flexiblen Körpers 44g angeordnet sein, der dann nur als Träger für die Elektroden 30g, 32g und die Abstandshalter 36g dient. Die mit biokompatiblem Material umhüllte Aufnehmerspule 46g paßt in eine schräge Durchbrechung 18g. Die Umhüllung der Aufnehmerspule 46g ist an ihren Enden mit vorspringenden Laschen 74 versehen, so daß die umhüllte Aufnehmerspule 46g ohne in den Hohlraum 16g durchzufallen wie ein Stöpsel in die betreffende Durchbrechung eingedrückt werden kann.

Bei der in Fig. 8c dargestellten Abwandlung der Elektrifizier-vorrichtung der Fig. 8b trägt der streifenförmige Körper 12h nur eine einzige Elektrode 30h und zwei beidseits von dieser angeordnete leistenförmige Abstandshalter 36h. Die zweite Elektrode wird durch die metallische Pfannenprothese gebildet, die über mindestens ein mit dem zweiten Ende der Aufnehmerspule 46h gekoppeltes Kontaktstück 76 kontaktiert ist. Das Kontaktstück 76 befindet sich am einen Ende der Umhüllung der Aufnehmerspule 46h.

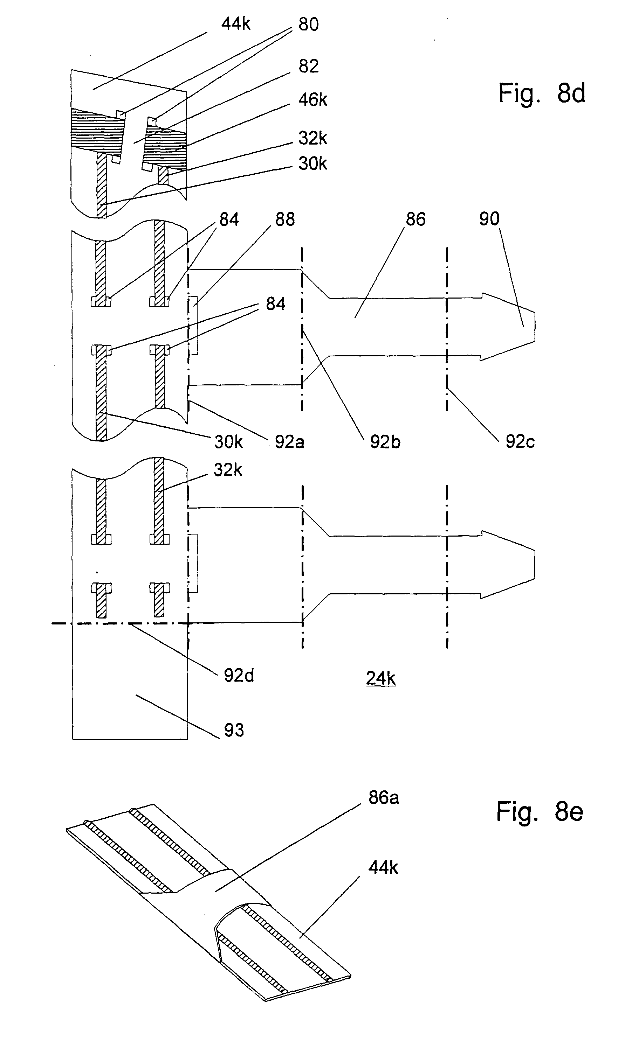

Die Figuren 8d und 8e zeigen eine weitere Ausführungsform einer Elektrifiziervorrichtung für eine Prothesenpfanne der in Fig. 8a dargestellten Art. Die Vorrichtung 24k hat einen dünnen, bandförmigen Träger 44k, der zur Vereinfachung der Zeichnung gerade und nicht, wie in den Fig. 8b und 8c gekrümmt dargestellt ist. Der Träger 44k hat am einen Ende zwei ausgestanzte, schlitzförmige Löcher 80, die einen Steg 82 bilden. Durch die Löcher 80 ist eine Aufnehmerspule 46k gesteckt. Der Träger 44k hat ferner in seiner Längsrichtung zwei Reihen von Lochpaaren 84, durch die streifenförmige Elektroden 30k, 32k gefädelt sind. Am einen Längsrand hat der Träger 44k mehrere integrale Laschen 86 mit pfeilförmigem Ende 90. Die Laschen 86 werden.an den strichpunktiert gezeichneten Linien 92a, 92b, 92c nach hinten geknickt und mit ihren Enden 90 durch Langlöcher 88 gesteckt. Ein Endstück (Abschlußlasche) 93 des Trägers 44k wird an den strichpunktiert gezeichneten Linie 92d nach vorne umgelegt, bevor das Ende der benachbarten Lasche 86 durch das zugehörige Langloch 88 gesteckt wird. Der Abstand zwischen den Knicklinien 92a und 92b entspricht im wesentlichen der Breite des bandförmigen Trägers 44k. Der Abstand zwischen den Knicklinien 92b und 92c ist jedoch etwas größer als die Breite des Trägers 44k, sodaß das betreffende Stück 86a (Fig. 8e) der Lasche 86 sich wölbt und als Abstandshalter wirkt.

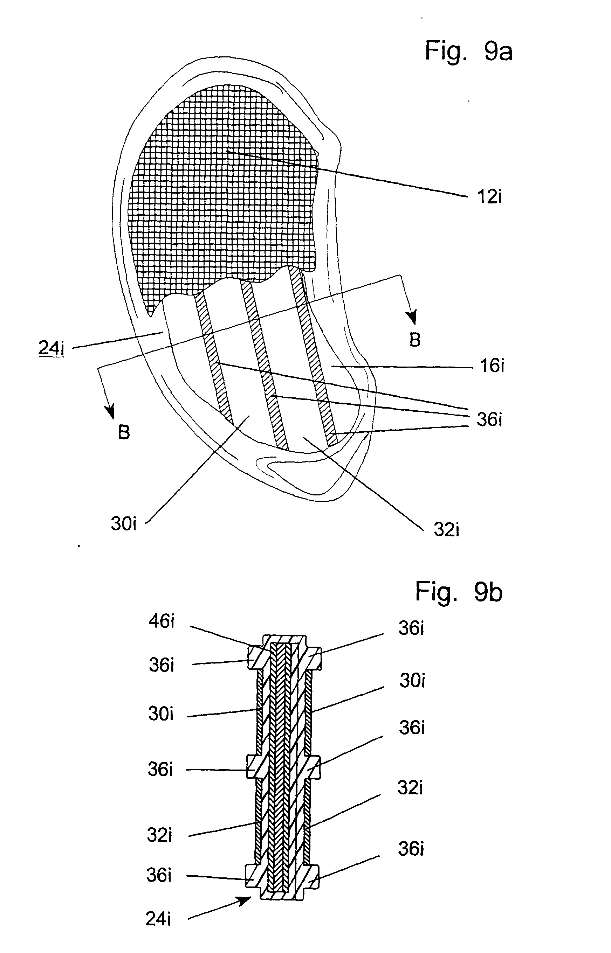

Fig. 9a und 9b zeigen ein Ausführungsbeispiel der vorliegenden Vorrichtung, das zur Förderung des Aufbaus von Binde- und Knorpelgewebes eines menschlichen Ohres dient. Die Vorrichtung hat hier einen Körper 12i in Form eines flachen Beutels aus netzförmigem gewebeverträglichen, ggf. resorbierbaren Material, das von Natur aus Durchbrechungen (Maschen) aufweist und in eine dem gewünschten Verwendungszweck angepaßte Form gebracht werden kann. Der netzförmige Körper 12i umschließt einen Raum 16i, in dem sich eine flache, flexible Elektrifiziervorrichtung 24i befindet, die eine flache, flexible Aufnehmerspule 46i mit einer biokompatiblen Umhüllung enthält und analog zu der gem. Fig. 8b und 8c ausgebildet sein kann. Die Umhüllung weist jedoch zweckmäßigerweise auf beiden Seiten Elektroden 30i, 32i und Abstandshalter 36i auf und ist in ihrer Form der des Implantatnetzbeutels angepaßt. Wenn der netzförmige Körper einer solchen Vorrichtung ganz oder teilweise aus einem elektrisch leitenden Material besteht, siehe die eingangs genannte DE-A-30 03 758, können die Elektroden 30i, 32i auf der Umhüllung der Aufnehmerspule 46i alle mit dem einen Anschluß der Aufnehmerspule 46i verbunden sein, während der beutelförmige Körper 12i als Gegenelektrode mit dem anderen Anschluß der Aufnehmerspule 46i verbunden ist.

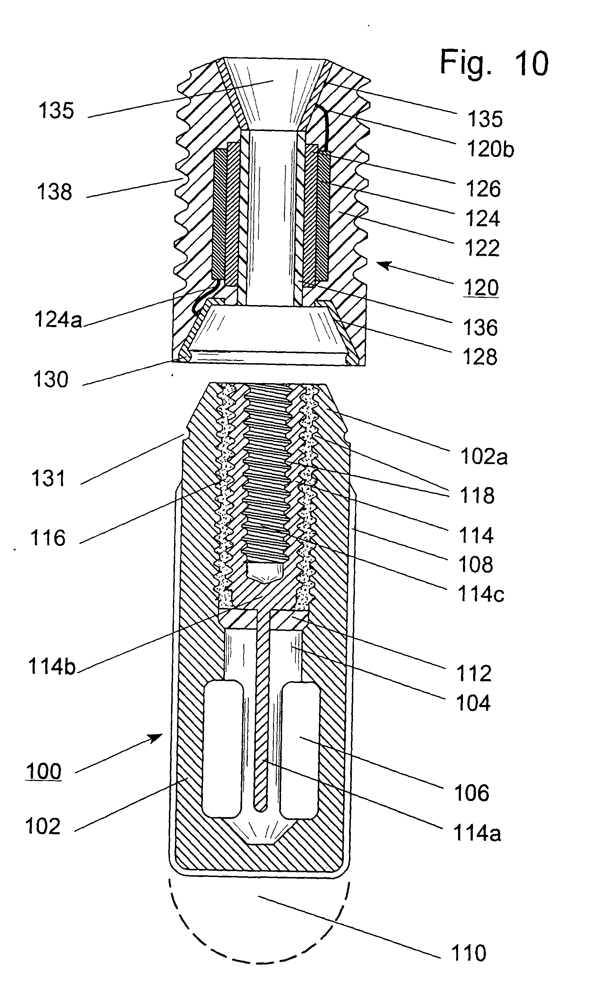

Fig. 10 zeigt eine weitere Ausführungsform eines als Träger für einen nicht dargestellten Kunstzahn geeigneten Dentalimplantats 100. Das Dentalimplantat hat einen hohlen, implantierbaren Schaft 102 aus einen elektrisch leitenden biokompatiblen Material, z. B. einem Metall wie Titan odereiner Kobalt-Chrom-Legierung. Der Schaft bildet einen Hohlraum 104, der im unteren Teil eine Anzahl von Fenstern oder Durchbrechungen 106 aufweist. Außen ist der Schaft mit einer Beschichtung 108 aus Hydroxylapatit überzogen. Das untere Ende kann abgerundet oder mit einer durchbrochenen, kalottenförmigen Kappe 110 versehen sein. Innen hat der Schaft 102 oberhalb der Durchbrechungen 106 eine ringförmige Schulter, die als Sitz für eine Ringscheibe 112 dient. Die Ringscheibe 112 bildet eine Halterung für eine Elektrode 114, die einen dünneren unteren stabförmigen Teil 114a und einen auf der Ringscheibe aufliegenden dickeren oberen Teil 114b mit einem Innengewinde 114c hat. Der Zwischenraum zwischen dem dickeren Teil 114b und der gegenüberliegenden Innenwand des Schaftes ist mit einem festen Isoliermaterial 116, z. B. Kunstharz oder Zement, ausgefüllt. Um eine feste Verbindung zu gewährleisten, sind die Außenseite des dickeren Elektrodenteils 114b und die gegenüberliegende Teil der Innenwand des Schaftes mit einem Gewinde 118 versehen oder anderweitig profiliert.

Das obere Ende 102a des Schaftes 102 ist konisch und bildet einen Sitz für eine aufgesetzte, gesonderte Aufnehmerspuleneinheit 120. Diese hat einen Kunststoffkörper 122, der eine mehrlagige Aufnehmerspule 124 umschließt, in der ein hohlzylinderförmiger Weicheisen-Magnetkern 126 angeordnet ist. Ein Anschluß 124a der Aufnehmerspule 124 ist mit einem kegelstumpfmantelförmigen Kontaktstück 128 verbunden, das in das untere Ende des Kunststoffkörpers 122 eingelassen ist. Es hat am unteren Ende einen ringförmigen Wulst 130, der im montierten Zustand in eine Ringnut 131 des Schaftes 102 eingreift und dadurch Kontakt mit dem oberen Ende 102a des Schaftes 102 macht. Der andere Anschluß 120b der Aufnehmerspule 124 ist mit einer trichterförmigen Metallhülse 134 verbunden, die in das obere Ende des Kunststoffkörpers 122 eingelassen ist. Durch den Kunststoffkörper 122 und den Magnetkern 126 verläuft ein axialer Kanal 135, der ein Isolierrohr 136 enthält. Außen weist der Kunststoffkörper ein Außengewinde 138 zum Aufschrauben eines Kunstzahnes auf.

Der nicht dargestellte Kunstzahn wird auf der Einheit 120 wird mittels einer nicht dargestellten Schraube, analog der Schraube 59 in Fig. 5, fixiert. Die Schraube reicht durch den Kanal 135 und wird in das Innengewinde 114c eingeschraubt, sodaß sie außer der mechanischen Verbindung von Kunstzahn, Einheit 120 und Schaft 102 auch eine elektrische Verbindung zwischen dem Anschluß 124 und der Elektrode 114 herstellt. Der Hohlraum 104 dient wie bei den vorangehenden Beispielen zur Aufnahme von Spongiosa u. dgl.

Generell kann der Körper der vorliegenden Vorrichtung, wenn er ganz oder teilweise aus einem elektrisch leitenden Material besteht, als zweite oder Gegenelektrode dienen, die dann mit der oder den inneren Elektroden eine Art von koaxialer Struktur bildet und einen im wesentlichen von innen nach außen fließenden Strom erzeugt.

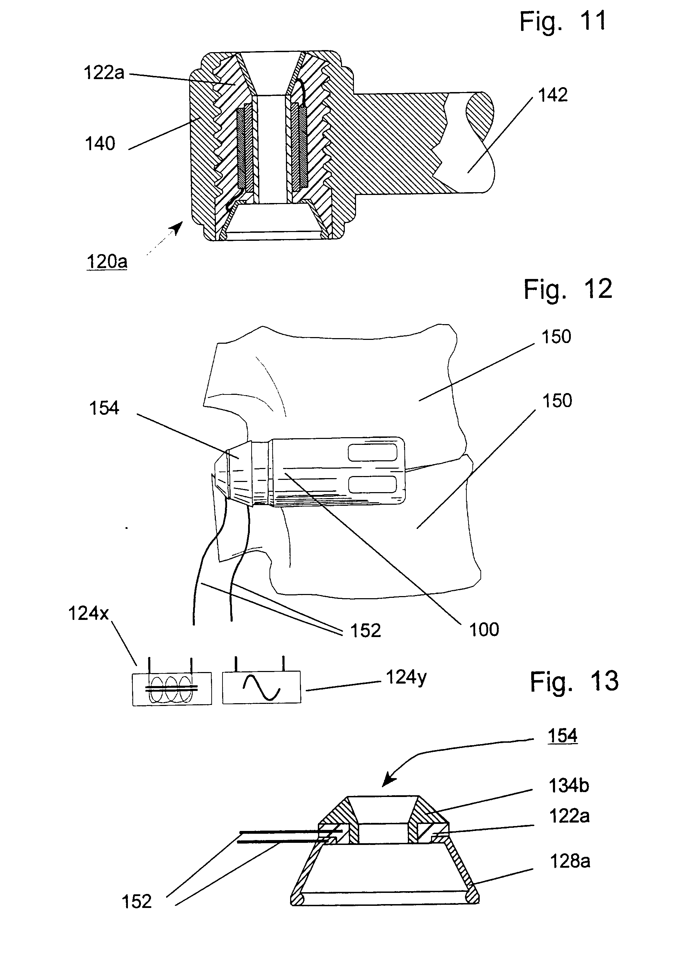

Fig. 11 zeigt eine Aufnehmerspuleneinheit 120a ähnlich der Einheit 120 gemäß Fig 10. Hier ist auf dem Außengewinde des Kunststoffkörpers 122a eine Gewindehülse 140 mit einer Verbindungsbrücke 142 befestigt.

Das in Fig. 10 dargestellte Implantat 100 kann auch mit einer räumlich getrennten, unabhängig implantierbaren Aufnehmerspule verwendet werden, z. B. zur Versteifung von zwei Lendenwirbeln 150, wie es in Fig. 12 gezeigt ist. Die Stromversorgung der Elektroden der Einheit 100 erfolgt durch eine räumlich entfernte Aufnehmerspule 124x oder eine andere implantierbare Stromquelle, z. B. einen Wechselstromgenerator 124y, die über flexible, isolierte Leitungen 152 und eine in Fig. 13 genauer dargestellte Anschlußkappe 154 mit dem Schaft 120 bzw. der Elektrode 114 (Fig. 10) gekoppelt ist.

Die Anschlußkappe 154 enthält ein trichterförmiges Kontaktstück 128a entsprechend dem Kontaktstück 128 in Fig. 10, und ein zweites trichterförmiges Kontaktstück 134b. Die Kontaktstücke sind durch einen Isolierkörper 122a getrennt. Die Anschlußkappe wird durch eine nicht dargestellte Schraube mit der Einheit 120 verbunden, wie es bezüglich der Einheit 120 anhand von Fig. 10 erläutert wurde.

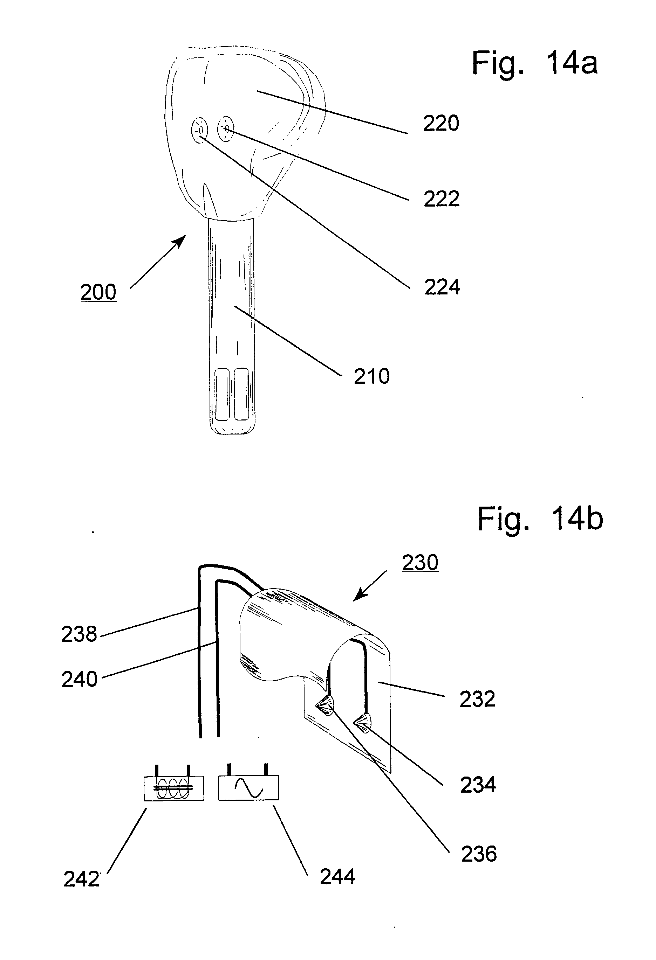

Fig. 14a zeigt ein Dentalimplantat 200, das einen implantierbaren ersten Teil in Form eines Schaftes 210 und einen bei implantiertem Schaft noch von außen zugänglichen zweiten Teil in Form eines Kunstzahnes 220 enthält. Der Schaft 210 kann im Aufbau dem gemäß Fig. 10 entsprechen, hat also einen hohlen Körper mit elektrisch leitfähiger Oberfläche und Durchbrechungen sowie mindestens eine Innenelektrode. Das Implantat 200 enthält jedoch keine Aufnehmerspule oder andere Spannungsquelle, vielmehr weist der von außen zugängliche zweite Teil, also hier der aufgeschraubte Kunstzahn 220 eine in Fig. 14a nicht dargestellte Anschlußvorrichtung der in Verbindung mit Fig. 13 erläuterten Art auf, deren Stromzuführungsleitungen in trichterförmigen Kontaktstücken 222, 224 enden, die an einer kosmetisch akzeptablen Stelle, z. B. der Rückseite des Kunstzahnes 220 in dessen Oberfläche eingelassen sind.

Die Stromversorgung der Elektroden (Schaftkörper, Innenelektrode) des Implantats erfolgt mit Hilfe einer in Fig. 14b dargestellten Klammer 230, die einen Bügel 232 aus nicht leitendem Material, wie Kunststoff enthält. An der Innenseite des Bügels 232 sind zwei kegelförmige Kontaktstücke 234, 236 befestigt, die in die Kontaktstücke 222, 224 passen und über flexible Leitungen 238, 240 mit einer Quelle für eine niederfrequente Wechselspannung koppelbar sind, z. B. einer Sekundär- oder Aufnehmerspule 242 oder einem Funktionsgenerator 244 oder anderen Wechselspannungsquelle. Hier ist also die Spannungsquelle extern, vom Implantat völlig getrennt und wird nur temporär an das Impantat angeschlossen.

Die Anschlüsse für die externe Stromquelle können auch an anderen Stellen angeordnet sein, z. B. am Schaft. Die Elektroden können auch mit einer internen Aufnehmerspule gekoppelt sein.

Auch bei der Vorrichtung gemäß Fig. 9a kann anstelle der Aufnehmerspule 46i - analog zu Fig. 12, 14a, 14b - eine getrennte, implantierbare oder externe Wechselspannungsquelle verwendet werden.

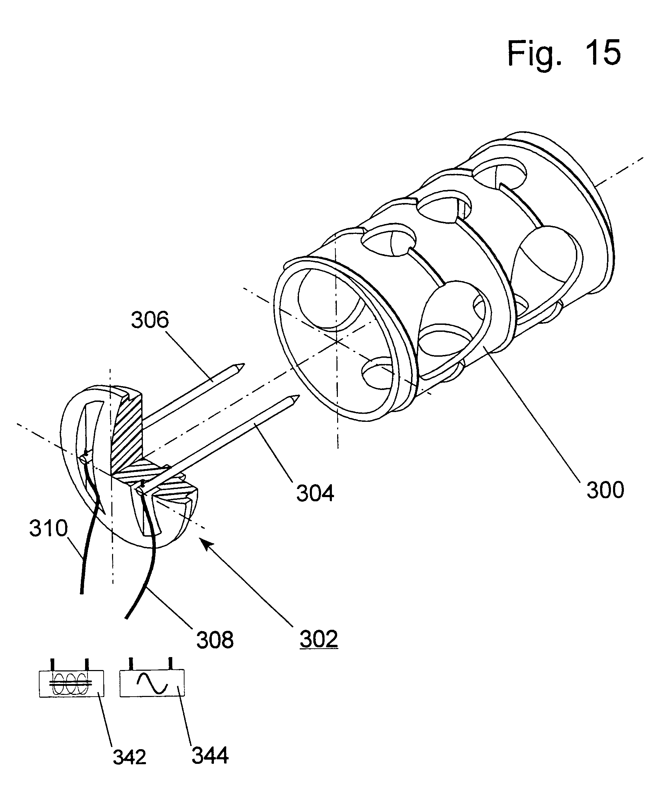

Fig. 15 zeigt eine ähnliche Vorrichtung wie Fig. 12 und 13. Sie enthält einen durchbrochenen zylindrischen Körper aus Titan, und eine Verschlußkappe 302 aus PTFE. Die Verschlußkappe haltert zwei axial verlaufende, stiftartige Elektroden 304, 306 mit spitzen Enden, die mit flexiblen, isolierten Leitungen 308, 310 zum Anschluß der Elektroden an eine implantierte Aufnehmerspule 342 oder eine andere Wechselspannungsquelle 344 verbunden sind. Wenn der Körper 300 zwischen zwei Rückenwirbeln implantiert und mit Spongiosa gefüllt ist, wird das offene Ende des Körpers mit der Verschlußkappe 302 verschlossen, wobei die Elekroden in die Spongiosa im Körper eindringen.

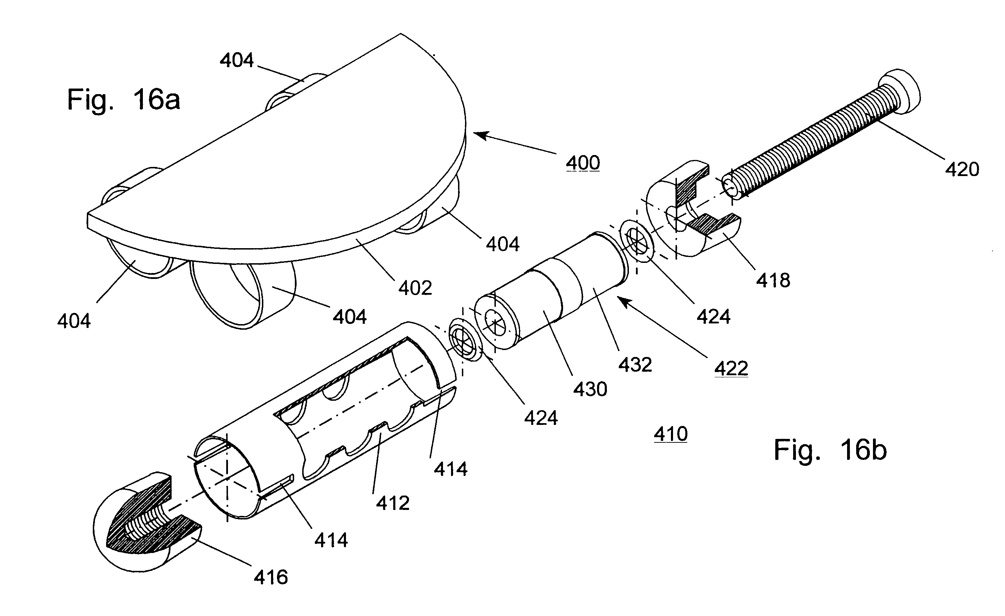

Fig. 16a zeigt den Tibia-Teil der aus der Zeitschrift OSTEOLOGIE (l. c.) bekannten Kniegelenkprothese. Er hat einen halbkreisförmigen Körper 402, an dessen Unterseite vier zylinderische Ringe 404 angebracht sind. Der Körper wird durch Spreizhülsen oder Dübel 410 (Fig. 16b und 16c), die durch die Ringe 404 gesteckt werden, im Tibiakopf befestigt.

Der in Fig. 16b und 16c dargestellte Dübel 410 enthält eine durchbrochene, zylindrische Hülse 412, die an den Enden axiale Schlitze hat. Die Enden können schwach konisch erweitert sein, wie es in Fig. 16c dargestellt ist. Der Dübel enthält ferner ein vorderes und ein hinteres, leicht konisches Endstück 416 bzw. 418, die mittels einer Schraube 420 in die Enden der Hülse 412 gedrückt werden können und dann die geschlitzten Enden spreizen.

In der Hülse 412 ist eine Vorrichtung 422 zum Stimulieren des Gewebewachstums angeordnet. Sie sitzt im montierten Zustand des Dübels 410 auf der Schraube 420 und wird durch zwei Ringscheiben 424 zentriert. Die Vorrichtung 422 enthält eine Aufnehmerspule 426, die auf einen rohrförmigen Magnetkern gewickelt ist. Die Enden der Spule 426 sind mit ringförmigen Elektroden 430, 432 gekoppelt. Zwischen den Elektroden 430, 432 und der Innenwand der Hülse 412 verbleibt ein Zwischenraum, in den, - stimuliert durch das elektrische Feld bzw. den elektrischen Strom zwischen den Elektroden, - Gewebe hineinwachsen kann. Der Dübel 410 kann auch als Prothesenschaft dienen, wobei dann das Endstück 418 z. B. als Halterung für einen Hüftgelenkkopf ausgebildet sein kann.

Anstelle von zwei rohrförmigen Elektroden können auch Elektroden in Form der Hälften oder Viertel usw. eines in Längsrichtung geteilten Zylinders verwendet werden.

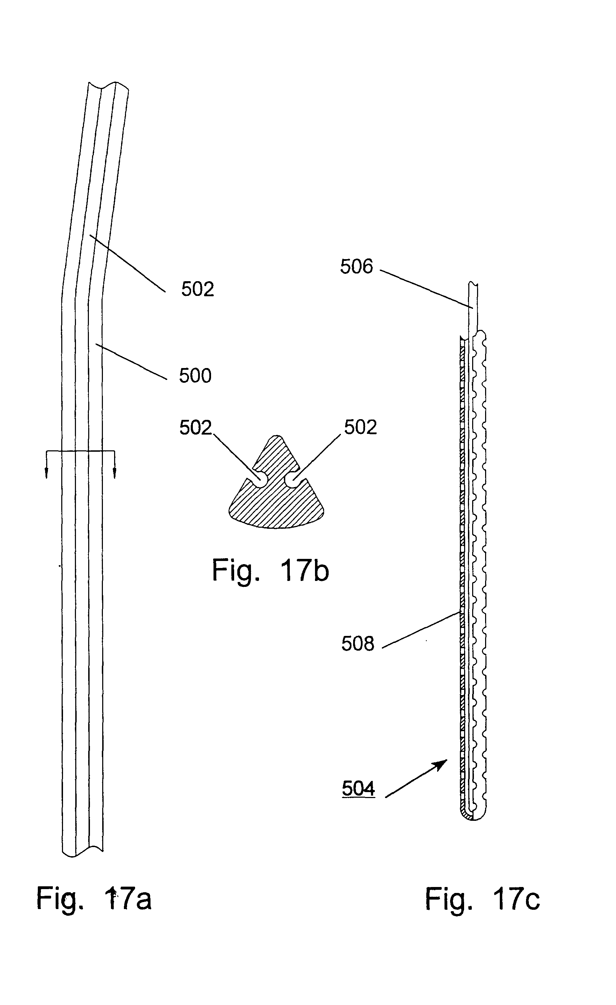

Fig 17a zeigt einen Teil eines Marknagels 500, der, wie der Querschnitt in Fig. 17b zeigt, Längsnuten 502 aufweist, die an ihrer Mündung etwas verengt sind. In den Längsnuten 502 wird eine Vorrichtung 504 eindrückt oder eingeschoben. Die Vorrichtung 504 enthält eine drahtförmige Elektrode 506, die mit Abstand von einem perforierten oder aus grobporigen Sintermaterial bestehenden Schlauch 508 umgeben ist. Der Schlauch kann aus PTFE bestehen und die Perforationen können mittels eines Lasers hergestellt werden. Der Marknagel 500 und die Elektrode 506 werden mit einer Aufnehmerspule (nicht dargestellt) verbunden, so daß zwischen der Elektrode und dem Marknagel 500 ein elektrisches Feld entsteht, das das Gewebewachstum fördert. Auch hier kann also Gewebe durch die schlitzförmige Öffnung der Nut in das aus den Perforationen oder Poren gebildeten Raum zwischen der Innenwand der Nut des Marknagels und der internen Elektrode hinein wachsen.

Alternativ können zwei oder mehr Vorrichtungen 504 in einer entsprechenden Anzahl von Nuten vorgesehen sein und die niederfrequente Wechselspannung zwischen die Elektroden 506 dieser Vorrichtungen 504 gelegt werden.

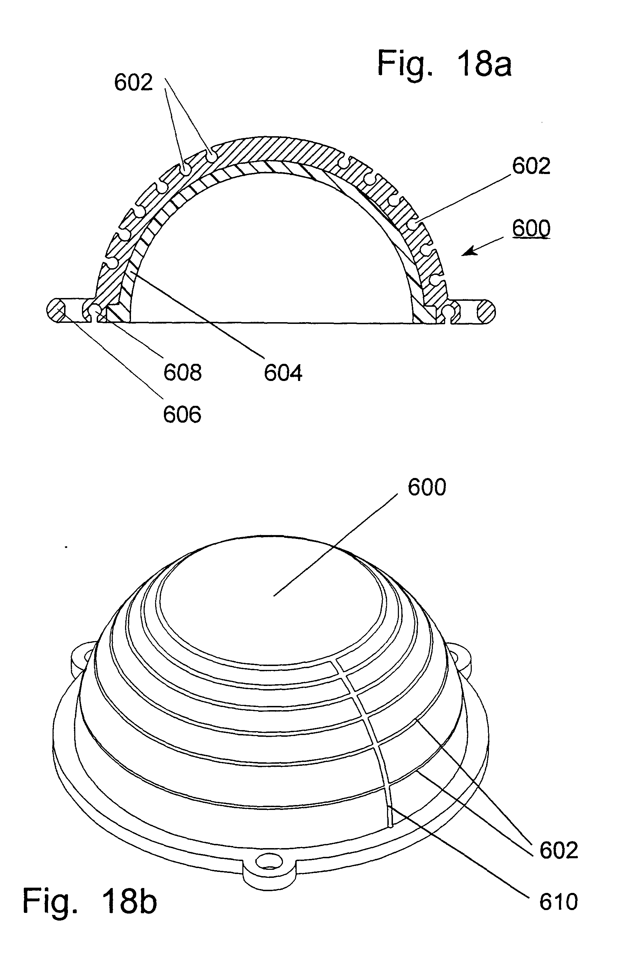

Die Vorrichtung 504 kann auch bei anderen Implantaten verwendet werden, z. B. bei einer Hüftgelenkkopfprothese, deren Schaft mit einer oder mehreren Nuten versehen ist oder einer Hüftgelenkpfannenprothese, wie die Fig. 18a und 18b zeigen. Die dort dargestellte Hüftgelenkpfannenprothese enthält einen kalottenförmigen Körper 600 aus Titan oder einem anderen gewebeverträglichen Metall. Außen weist der Körper 600 eine Anzahl von ringförmigen Nuten 602 mit schlitzförmigen Öffnungen entsprechend den Nuten 502 in Fig. 17b, auf, innen ist er mit einer Schale 604 aus PTFE ausgekleidet. Am Rand sind mehrere Augen 606 vorgesehen, die Löcher zur Befestigung der Prothese am Hüftknochen bilden. Der Rand enthält eine Nut 608 für eine nicht dargestellte Aufnehmerspule. Die Nut 608 steht über ein Loch mit einer meridionalen Nut 610 in Verbindung, die die Nuten 602 schneidet.

In die Nuten 602 werden Vorrichtungen 504 der in Fig. 17c dargestellten Art eingeführt, deren Elektroden 506 über Verbindungsleitungen, die in der Nut 610 verlaufen, mit der Aufnehmerspule verbunden werden. Wie bei Fig. 17c bilden die Löcher des isolierenden Schlauches der Vorrichtung 504 Hohlräume, die von der Oberfläche der Elektrode zur Öffnung der Nut führen. Die Elektroden in den Nuten 602 können abwechselnd mit gegenpoligen Anschlüssen der Aufnehmerspule verbunden werden oder auch alle mit dem einen Anschluß, wobei dann der Körper 600 als Gegenelektrode geschaltet wird. Der Körper kann auch eine oder mehrere spiralförmige Nuten analog einem ein- oder mehrgängigen Gewinde für entsprechende Vorrichtungen 506 aufweisen.

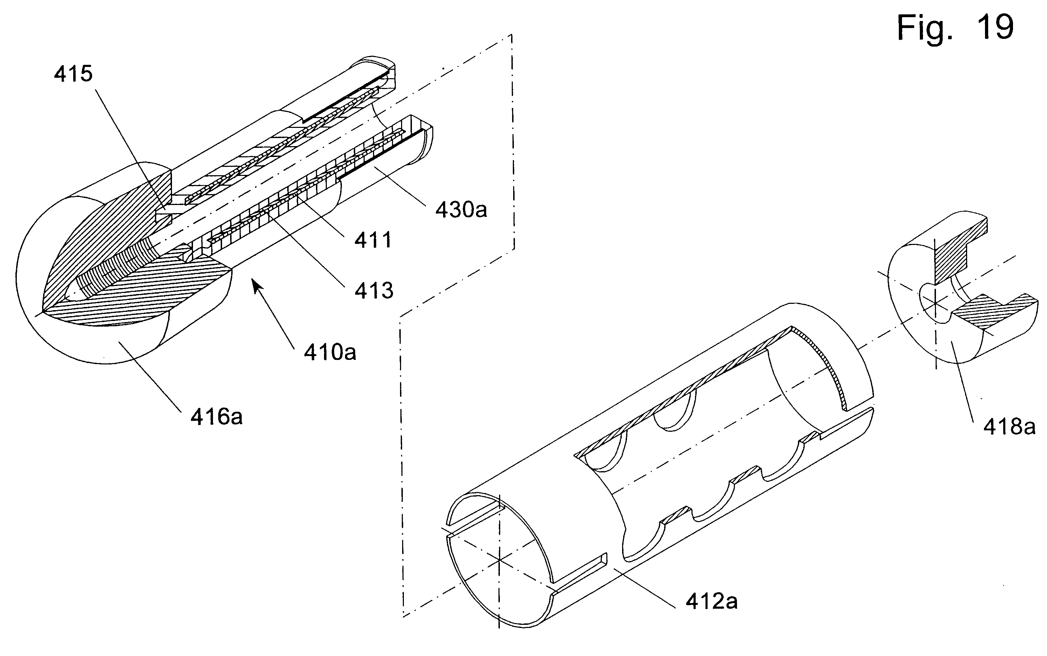

Fig. 19 zeigt einen Elektro-Dübel ähnlich dem gemäß Fig. 16b. Entsprechende Teile sind daher mit gleichen Bezugszahlen, denen ein "a" angefügt ist, bezeichnet und die Erläuterung wird auf die Unterschiede beschränkt.

Die Vorrichtung 410a des Dübels gemäß Fig. 19 enthält nur eine einzige rohrförmige Elektrode 430a, die mit einem ersten Anschluß einer Aufnehmerspule 411 gekoppelt ist. Die Aufnehmerspule 411 sitzt auf einem rohrförmigen Magnetkern 413, beide sind von dem aus PTFE bestehenden rohrförmigen Körper der Vorrichtung 410a umschlossen, der in einer Ringnut an der rückwärtigen Stirnseite des Endstücks 410a sitzt. Der zweite Anschluß der Aufnehmerspule ist über eine Kontaktiervorrichtung 415 elektrisch mit dem aus Metall bestehenden Endstück 416a verbunden, das zusammen mit dem rohrförmigen, gelochten Metallteil 412a und dem ebenfalls aus Metall bestehenden Endstück 418a als Gegenelektrode dient. Im übrigen entspricht der Dübel gemäß Fig. 19 dem gemäß Fig. 16b

## Patentansprüche

1. Implantierbare Vorrichtung mit einem ihrer medizinischen Funktion entsprechenden, aus biokompatiblen Material bestehenden Körper [12], welcher mindestens einen inneren Hohlraum [16] bildet, der durch mindestens eine Öffnung [18] mit der Umgebung des Körpers in Verbindung steht, und mit mindestens zwei Elektroden, welche Anschlüsse zur Zuführung einer niederfrequenten elektrischen Wechselspannung aufweisen und von denen mindestens eine [30] innerhalb des Hohlraums [16] angeordnet ist, **dadurch gekennzeichnet, daß** die mindestens eine Elektrode [30] innerhalb des Hohlraums [16] so angeordnet ist, daß sie von der inneren Begrenzung des Hohlraums mit Abstand umgeben wird und zwischen dieser Elektrode [30] und der inneren Begrenzung des Hohlraums ein Zwischenraum verbleibt, in den Gewebe von außen durch die Öffnung(en) [18] hineinwachsen kann.

2. Vorrichtung nach Anspruch 1, **dadurch gekennzeichnet, daß** mindestens eine zweite Elektrode [32] im Hohlraum [16] angeordnet ist.

3. Vorrichtung nach Anspruch 1 oder 2, **gekennzeichnet durch** eine Aufnehmerspule [46], die mit den Elektroden [30, 32] elektrisch gekoppelt ist.

4. Vorrichtung nach Anspruch 1, bei der der Körper [12] einen hohlen Gelenkprothesenschaft bildet, der seitliche Durchbrechungen [18] hat und an einem halsseitigen Ende ein Gewindeloch [20] aufweist, **gekennzeichnet durch** eine in das Gewindeloch [20] einsetzbare Elektrifizier-Vorrichtung [24], die einen in den Hohlraum [16] reichenden stabförmigen Schaft [28] hat, der die mindestens eine Elektrode [30] trägt und eine Aufnehmerspule [46] enthält.

5. Vorrichtung nach Anspruch 4, **dadurch gekennzeichnet, daß** die Elektrifiziervorrichtung [24a, Fig. 2] eine in das Gewindeloch [20] passende Schraube [26] enthält, an der der Schaft [28a] der Elektrifizierrvorrichtung befestigt ist, und daß zwischen der Schraube und dem sich an diese anschließenden Teil des Schaftes eine Sollbruchstelle [33] vorgesehen ist.

6. Vorrichtung nach Anspruch 1, bei der der Körper die Form einer Hüftgelenkpfannen-Prothese hat und einen ringförmigen Hohlraum bildet, **dadurch gekennzeichnet, daß** der Hohlraum eine Elektrifiziervorrichtung [24f] mit einem flexiblen, bandförmigen Körper [44f] enthält, welcher mindestens eine sich in Umfangsrichtung des Hohlraumes erstreckende Elektrode [30f] trägt.

7. Vorrichtung nach Anspruch 1 oder 2, **dadurch gekennzeichnet. daß** der Körper [12i] die Form eines Beutels aus netzförmigem Material hat.

8. Vorrichtung nach Anspruch 7, **dadurch gekennzeichnet, daß** der Körper eine flache, flexible Aufnehmerspule enthält, die mit einer Umhüllung aus einem biokompatiblen Material versehen ist, auf der Elektroden [30i, 32i] und Abstandshalter [36i] angeordnet sind [Fig. 9a, b].

9. Vorrichtung nach Anspruch 1, welche einen implantier baren hohlen Schaft [200] und einen Kunstzahn [220] enthält, **dadurch gekennzeichnet, daß** der Kunstzahn [220] Anschlüsse [222] für eine externe Stromquelle aufweist und daß der hohle Schaft Durchbrechungen aufweist und die mindestnes eine Elektrode enthalt, die mit einem der Anschlüsse gekoppelt ist [Fig. 14a, 14b].

10. Vorrichtung nach Anspruch 1, **dadurch gekennzeichnet, daß** der aus elektrisch leitfähigem Material bestehende Körper [500, 600] mindestens eine Nut [502, 602] aufweist, die eine Öffnung hat, daß in der Nut eine Elektrode [506] angeordnet ist, und daß die Elektrode von einem durchbrochenen, elektrisch isolierenden Gebilde [508] umgeben ist, das Löcher hat, die von der Elektrode zur Öffnung der Nut führen.

## Claims

1. An implantable device with a body (12) suitable for its medical function and comprising biocompatible material, which body forms at least one interior cavity (16) that communicates through at least one opening (18) with the surroundings of the body, and having at least two electrodes which have terminals for supplying a low-frequency electrical alternating voltage and at least one (30) of which is located inside the cavity (16), **characterized in that** the at least one electrode (30) is located inside the cavity (16) in such a way that it is surrounded in spaced-apart fashion by the inner boundary of the cavity, and an interstice remains between this electrode (30) and the inner boundary of the cavity, into which interstice tissue can grow from outside inward through the opening or openings (18).

2. The device as claimed in claim 1, **characterized in that** at least one second electrode (32) is disposed in the cavity (16).

3. The device as claimed in claim 1 or 2, **characterized by** a pickup coil (46) that is electrically coupled to the electrodes (30, 32).

4. The device as claimed in claim 1, in which the body (12) forms a hollow joint prosthesis shaft that has lateral apertures (18) and has a threaded hole (20) on an end toward the neck, **characterized by** an electrifier (24) which is adapted to be inserted into the threaded hole (20) and has a rodlike shaft (28) extending into the cavity (16), which shaft carries the at least one electrode (30) and contains a pickup coil (46).

5. The device as claimed in claim 4, **characterized in that** the electrifier (24a, Fig. 2) contains a screw (26) that fits into the threaded hole (20) and to which the shaft (28a) of the electrifier is secured, and that a rated breaking point (33) is provided between the screw and the portion of the shaft adjoining it.

6. The device as claimed in claim 1, in which the body takes the form of an acetabulum prosthesis and forms an annular cavity, **characterized in that** the cavity contains an electrifier (24f) with a flexible bandlike body (44f) that carries at least one electrode (30f) extending circumferentially of the cavity.

7. The device as claimed in claim 1 or 2, **characterized in that** the body (12i) takes the form of a bag of meshlike material.

8. The device of claim 7, **characterized in that** the body contains a flat, flexible pickup coil which is provided with an envelope of a biocompatible material on which electrodes (30i, 32i) and spacers (36i) are located (Figs. 9a, b).

9. The device of claim 1, which contains an implantable hollow shaft (200) and an artificial tooth (220), charaterized in that the artificial tooth (220) has terminals (222) for an external current source, and that the hollow shaft has apertures and contains the at least one electrode, which is coupled to one of the terminals (Figs. 14a, 14b).

10. The device as claimed in claim 1, **characterized in that** the body (500, 600) comprising electrically conductive material has at least one groove (502, 602), which has an opening; that an electrode (506) is located in the groove; and that the electrode is surrounded by a perforated, electrically insulating structure (508) that has holes which lead from the electrode to the opening of the groove.

## Revendications

1. Dispositif implantable comportant un corps [12] qui est en conformité avec sa fonction médicale et consiste en matériau biocompatible, ledit corps formant au moins un espace creux interne [16] qui est en communication avec l'evironnement du corps par au moins une orifice [18], et au moins deux électrodes qui comportent des connexions d'alimentation en courant alternatif à basse fréquence dont au moins une [30] est disposée à l'intérieur de l'espace creux [16], **caractérisé en ce que** ladite au moins une électrode [30] est disposée à l'intérieur de l'espace creux [16] de façon à être entourée à distance par la périphérie interne de l'espace creux, et ladite électrode [30] et ladite limitation interne de l'espace creux forment entre elles une lacune qui est apte à être remplie par tissu croissant de l'éxtérieur vers l'intérieur à travers la/les orifice(s) [18].

2. Dispositif suivant la revendication 1, **caractérisé en ce que** au moins une deuxième électrode [32] est disposée dans l'espace creux [16].

3. Dispositif suivant la revendication 1 ou 2, **caractérisé par** une bobine à réception [46] qui est reliée électriquement aux électrodes [30, 32].

4. Dispositif suivant la revendication 1, dans lequel le corps [12] forme une tige creux pour une prothèse articulaire, ledit tige comportant des découpures [18] et, à son extrémité du col, un trou taraudé [20], **caractérisé par** un dispositif d'électrification [24] apte à être disposé dans le trou taraudé [20], ledit dispositif comportant un tige [28] en forme de barre qui s'étend dans l'espace creux et comprend la bobine à réception [46].

5. Dispositif suivant la revendication 4, **caractérisé en ce que** le dispositif d'électrification [24a, Fig. 2] comprend une vis qui est apte à être introduit dans le trou taraudé [20] et à laquelle est reliée la tige [28a] du dispositif d'électrification, et **en ce que** entre la vis et la partie adjacent de la tige est prévue un point destiné à la rupture [33].

6. Dispositif suivant la revendication 1, dans lequel le corps est en forme d'une prothèse à cavité articulaire de la hanche et forme une espace creux annulaire, **caractérisé en ce que** l'espace creux comprend un dispositif d'électrification [24f] ayant un corps [44f] flexible en forme de bande et portant au moins une électrode [30f] qui s'étend dans la direction circonférentielle de l'espace creux.

7. Dispositif suivant la revendication 1 ou 2, **caractérisé en ce que** le corps [12i] est en forme d'un sachet consistant en matériau en forme de réseau.

8. Dispositif suivant la revendication 7, **caractérisé en ce que** le corps comprend une bobine à réception plate et flexible qui comporte une enveloppe d'un matériau biocompatible sur laquelle sont disposés des électrodes [30i, 32i] et des écarteurs [36i] [Fig. 9a, b].

9. Dispositif suivant la revendication 1, qui comprend un tige creux implantable [200] et un dent artificiel [220], **caractérisé en ce que** le dent artificiel [220] comporte des connexions [222] pour une source de courant externe, et **en ce que** le tige creux comporte de découpures et contient la au moins une électrode étant raccordée avec l'une des connexions [Fig. 14a, 14b].

10. Dispositif suivant la revendication 1, **caractérisé en ce que** le corps [500, 600] consistant en matériau électroconducteur comporte au moins une rainure [502, 602] ayant une orifice, **en ce que** une électrode [506] est disposée dans la rainure, et **en ce que** ladite électrode est entourée par un produit electro-isolateur [508] ayant des trous s'etendant à partir de l'électrode jusqu'à l'ouverture de la rainure.
